(19) Europäisches Patentamt
European Patent Office
Office européen des brevets

(11) Veröffentlichungsnummer: **0 381 041 A1**

(12) # EUROPÄISCHE PATENTANMELDUNG

(21) Anmeldenummer: **90101450.6**

(22) Anmeldetag: **25.01.90**

(51) Int. Cl.[5]: **C07C 317/22, C07C 315/00**

(30) Priorität: **01.02.89 DE 3902897**
**24.07.89 DE 3924395**

(43) Veröffentlichungstag der Anmeldung:
**08.08.90 Patentblatt 90/32**

(84) Benannte Vertragsstaaten:
**BE DE FR GB IT NL**

(71) Anmelder: **BASF Aktiengesellschaft**
**Carl-Bosch-Strasse 38**
**D-6700 Ludwigshafen(DE)**

(72) Erfinder: **Stumpp, Michael, Dr.**
**An der Marlach 13**
**D-6705 Deidesheim(DE)**
Erfinder: **Neumann, Peter, Dr.**
**Poststrasse 28**
**D-6800 Mannheim 31(DE)**
Erfinder: **Hupfeld, Bernd, Dr.**
**Mausbergweg 6**
**D-6720 Speyer(DE)**
Erfinder: **Reichelt, Helmut, Dr.**
**Johann-Gottlieb-Fichte-Strasse 56**
**D-6730 Neustadt(DE)**

(54) **Verfahren zur Herstellung von Bis-(4-hydroxyphenyl)-sulfon.**

(57) Verfahren zur Herstellung von Bis-(4-hydroxyphenyl)-sulfon durch Umsetzung von Phenol mit Schwefelsäure bei Temperaturen von 140 bis 230° C, indem man Phenol und Schwefelsäure im Molverhältnis von 1:1 bis 25:1 einsetzt und die Umsetzung in Abwesenheit eines inerten Lösungsmittels durchführt.

EP 0 381 041 A1

Xerox Copy Centre

## Verfahren zur Herstellung von Bis-(4-hydroxyphenyl)-sulfon

Die vorliegende Erfindung betrifft ein Verfahren zur Herstellung von Bis-(4-hydroxyphenyl)-sulfon durch Umsetzung von Phenol mit Schwefelsäure in Abwesenheit eines inerten Lösungsmittels.

Bis-(4-hydroxyphenyl)-sulfon ist für die Herstellung von Fasern, Harzen und hochtemperaturbeständigen Kunststoffen (z. B. Polyethersulfone) von großem wirtschaftlichen Interesse. Da die Eigenschaften der aus Bis-(4-hydroxyphenyl)-sulfon hergestellten Polymeren außerordentlich stark vom Reinheitsgrad der Monomeren abhängen, sind selektive Syntheseverfahren für Bis-(4-hydroxyphenyl)-sulfon gefragt.

Aus der DE-A-27 08 388 und der EP-A-220 004 sind Verfahren bekannt, bei denen die Umsetzung von Phenol mit Schwefelsäure in Gegenwart eines inerten Lösungsmittels bei Temperaturen von 150 bis 220 °C zu Bis-(4-hydroxyphenyl)-sulfon führt. Die Ausbeute und die Reinheit des Bis-(4-hydroxyphenyl)-sulfons wird dadurch erhöht, daß das Lösungsmittel kontinuierlich aus der Reaktion durch Destillation entfernt wird oder nach im wesentlichen beendeter Umsetzung abdestilliert wird, wobei es wesentlich ist, daß die Destillation im Temperaturbereich von 160 bis 200 °C vorgenommen wird, da nur dann eine Isomerisierung des 2,4′-Isomeren zum Bis-(4-hydroxyphenyl)-sulfon erreicht wird.

Der Erfindung lag die Aufgabe zugrunde, ein verbessertes Verfahren zur Herstellung von Bis-(4-hydroxyphenyl)-sulfon aus Phenol und Schwefelsäure zu entwickeln.

Demgemäß wurde ein verbessertes Verfahren zur Herstellung von Bis-(4-hydroxyphenyl)-sulfon durch Umsetzung von Phenol mit Schwefelsäure bei Temperaturen von 140 bis 230 °C gefunden, welches dadurch gekennzeichnet ist, daß man Phenol und Schwefelsäure im Molverhältnis von 1 : 1 bis 25 : 1 einsetzt und in Abwesenheit eines inerten Lösungsmittels arbeitet.

Sulfonierungsreaktionen (A), wie auch die Sulfonbildung (B) sind reversible Gleichgewichtsreaktionen. Zwischen den beiden Isomeren Bis-(4-hydroxyphenyl)-sulfon und 2-Hydroxy-4-hydroxyphenylsulfon besteht ein thermodynamisches Gleichgewicht (C). Dies zeigen die folgenden Reaktionsgleichungen:

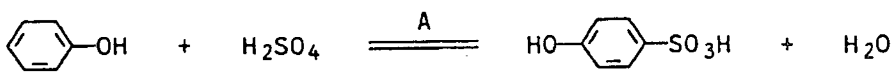


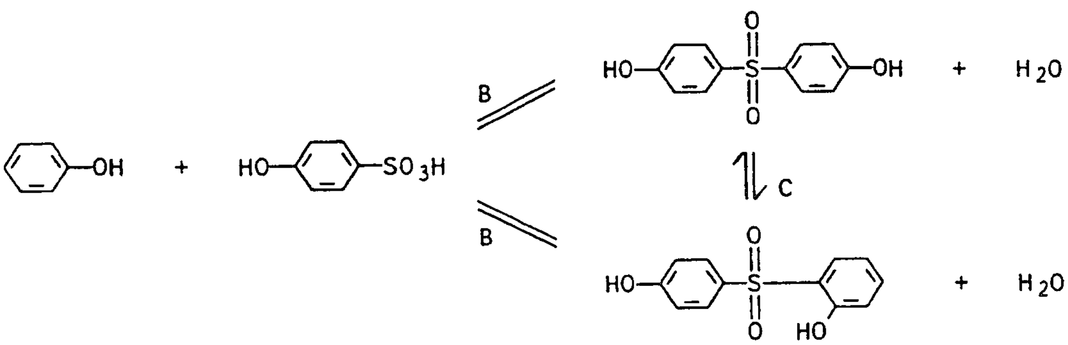


Sowohl das Sulfonierungsgleichgewicht (A), als auch das Sulfonbildungsgleichgewicht B kann bekannterweise durch Entfernung des Reaktionswassers aus dem Reaktionsmedium nahezu vollständig auf die Seite der Produkte verschoben werden. Einen Einfluß auf die Isomerenverteilung wird durch diese Verfahrensweise jedoch nicht erreicht. Wie aus der DE-A-27 08 388 bekannt, wird das thermodynamische Gleichgewicht zwischen den beiden Isomeren dadurch vorteilhafterweise beeinflußt, daß ein systemfremdes Lösungsmittel, das in der Lage ist, das unerwünschte Isomere besser zu lösen, als das erwünschte Bis-(4-hydroxyphenyl)-sulfon, während der Reaktion oder nach im wesentlichen beendeter Umsetzung durch Abdestillation bei 160 bis 200 °C entfernt wird.

Es wurde nun ein Verfahren zur Herstellung von Bis-(4-hydroxyphenyl)-sulfon durch Umsetzung von Phenol mit Schwefelsäure bei Temperaturen von 140 bis 230 °C gefunden, das dadurch gekennzeichnet ist, daß man Phenol und Schwefelsäure im Molverhältnis von 1:1 bis 25:1 einsetzt und die Umsetzung in Abwesenheit eines inerten Lösungsmittels durchführt.

Das erfindungsgemäße Verfahren kann wie folgt durchgeführt werden:

Die Komponenten Schwefelsäure und Phenol können gemeinsam vorgelegt und dann auf die Reaktionstemperatur von 140 bis 230 °C gebracht werden. Vorteilhaft wird die Schwefelsäure dem Phenol

kontinuierlich bzw. portionsweise im Verlauf der Reaktion, besonders vorteilhaft nach der Aufheizphase, zudosiert. Es empfiehlt sich eine Lewissäure, wie Borsäure oder Trifluormethansulfonsäure mitzuverwenden.

Das Molverhältnis von Phenol zu Schwefelsäure beträgt 1:1 bis 25:1, vorzugsweise 2,1:1 bis 10:1, besonders bevorzugt 3:1 bis 7:1; größere Mengen sind auch möglich.

Das erfindungsgemäße Verfahren kann im chargenweisen Betrieb in Rührkesseln ausgeübt werden. Zur kontinuierlichen Durchführung des erfindungsgemäßen Verfahrens können vorteilhaft Rohr- oder Röhrenreaktoren verwendet werden. Die Verweilzeit der Reaktionslösung im Rohrreaktor kann dabei u.a. durch die Beschickung des Rohrreaktors mit Füllkörpern gesteuert werden. Bevorzugt ist dabei die Anwendung kugelförmiger Füllkörper, wie sie auch in Füllkörperkolonnen zur destillativen Auftrennung von Gemischen benutzt werden, beispielsweise Glaskugeln oder Kugeln aus gegenüber der Reaktionsmischung inerten Stählen. Der Durchmesser dieser Kugeln beträgt in der Regel 0,4 bis 10 mm, vorzugsweise 1 bis 5 mm. Zweckmäßigerweise werden Kugeln einer bestimmten Größe verwendet. Vorteilhafterweise werden die Reaktionsrohre mit Füllkörpern aus Materialien bepackt, welche die Sulfonbildung katalytisch beeinflussen und somit als Sulfonierungskatalysatoren wirken. Beispielsweise lassen sich die Ausbeute und die Selektivität der Umsetzung steigern, wenn gasinerte Borsäurekugeln oder von mit einer Säure, wie Phosphor-, Schwefel- oder Borsäure, beladenes Kieselgel als Füllkörper eingesetzt werden. Derart modifiziertes Kieselgel wird zweckmäßigerweise durch Imprägnieren von Kieselgel mit einer wäßrigen Lösung der betreffenden Säure und anschließende Trocknung hergestellt.

Ein Vorteil des erfindungsgemäßen Verfahrens besteht darin, daß das Reaktionswasser nicht notwendigerweise aus der Umsetzung ausgekreist werden muß, um einen, bezüglich der verwendeten Schwefelsäure, vollständigen Umsatz zu erzielen.

Bei der Umsetzung werden im allgemeinen Temperaturen von 140 bis 230 °C, vorzugsweise von 160 bis 200 °C und besonders bevorzugt von 170 bis 190 °C angewandt. Bei einer Reaktionstemperatur von weniger als 165 °C wird zweckmäßigerweise bei vermindertem Druck gearbeitet, um das Sieden des Phenols zu gewährleisten. Üblicherweise wird das Verfahren jedoch unter Atmosphärendruck ausgeübt.

Das bei der Sulfonierung von Phenol mit Schwefelsäure entstehende Wasser kann erfindungsgemäß allerdings auch mittels des in der Reaktionsmischung vorhandenen, überschüssigen Phenols laufend azeotrop aus der Umsetzung abdestilliert werden und das Phenol nach Phasenabscheidung des Wassers wieder in den Reaktor zurückgeführt werden. Nach der Auskreisung von in der Regel mindestens 50 %, besser jedoch von 80 bis 100 % der sich nach der Theorie bildenden Wassermenge, wird im allgemeinen kein Phenol mehr in die Reaktion zurückgeführt, sondern das Phenol nach und nach bei 160 bis 200 °C abdestilliert.

Die Reaktionszeit beträgt im chargenweisen Verfahren im allgemeinen 4 bis 6 Stunden und die zur Isomerisierung benötigte Zeit 1 bis 4 Stunden. Im kontinuierlichen Verfahren, also der Passage der Reaktanten durch einen Rohr- oder Röhrenreaktor, wird bei Verweilzeiten von 1 bis 3 Minuten ein Umsatz von bis zu 60 % - bezogen auf die eingesetzte Schwefelsäure - erzielt. Durch die kontinuierliche Rückführung des am Reaktorende austretenden Reaktionsgemisches auf die als Reaktor dienende Füllkörperkolonne und einer dadurch verlängerten, durchschnittlichen Verweilzeit von ungefähr 15 Minuten kann der Gesamtumsatz der eingesetzten Schwefelsäure auf 95 % gesteigert werden.

Das erhaltene Rohprodukt kann zwecks weiterer Reinigung in wäßriger Alkalilösung aufgenommen und gegebenenfalls nach Behandlung mit Aktivkohle und Filtration mit Säuren, z. B. Mineralsäuren, insbesondere Schwefelsäure bei einem pH-Wert von ca. 6 bis 7 wieder ausgefällt werden.

## Beispiele

### Beispiel 1

In einem 2 l-Glaskolben wurden 470,6 g (5,0 Mol) Phenol, 102,2 g (1,0 Mol) 96 %ige Schwefelsäure und 6,2 g (0,1 Mol) Borsäure plaziert und auf 180 bis 186 °C erhitzt. Es wurde solange bei dieser Temperatur Wasser ausgekreist und Phenol wieder zurückgeführt, bis kein Reaktionswasser mehr entstand. Anschließend wurde das überschüssige Phenol während 2 Stunden abdestilliert, wobei die Temperatur 160 °C nicht unterschritt. [Eine GC-Analyse des Rohgemisches ergab folgende Zusammensetzung: 5 % Phenol, 3 % isomere Sulfone und 92 % Bis-(4-hydroxyphenyl)-sulfon]. Zur Entfernung restlicher Phenolspuren wurde der Rückstand in wäßriger (10 Gew.%) Natriumhydroxidlösung gelöst und das Bis-(4-hydroxyphenyl)-sulfon durch Ansäuern mit Schwefelsäure ausgefällt. Nach Absaugen und Trocken erhielt man 212 g (0,85 Mol; 85 % Ausbeute) Bis-(4-hydroxyphenyl)-sulfon in einer Reinheit > 98 %.

Beispiel 2

In einem 2 l-Glaskolben wurden 470,6 g (5,0 Mol) Phenol und 6,2 g (0,1 Mol) Borsäure vorgelegt. Dazu wurden während Erhitzen auf 180 bis 186 °C 102,2 g (1,0 Mol) 96 %ige Schwefelsäure zugetropft. Nach dem Zutropfen wurde bei 180 bis 186 °C das Reaktionswasser ausgekreist. Dann wurde das Phenol während 2 Stunden bei 160 bis 200 °C abdestilliert. Eine Analyse des Rohproduktes ergab folgende Zusammensetzung: 3 % Phenol, 1 % isomere Sulfone und 97 % Bis-(4-hydroxyphenylsulfon). Zur weiteren Reinigung wurde, wie unter Beispiel 1 beschrieben, verfahren. Man erhielt 205 g 0,82 Mol; 82 % Ausbeute) Bis-(4-hydroxyphenyl)-sulfon in einer Reinheit von > 99 %.

Beispiel 3

In eine auf 180 °C beheizte, stehende und mit Glaskugeln (Durchmesser 4 mm) gefüllte Glasröhre (Länge 80 cm, Durchmesser 4 cm) wurden simultan 102 g (1,0 mol) Schwefelsäure 96 %ig und 546 g (6,0 mol) Phenol innerhalb einer halben Stunde zugetropft. Die Verweilzeit des Reaktionsgemisches in der Festbettkolonne betrug etwa 1 bis 3 Minuten. Das Reaktionsgemisch wurde am Auslauf des Reaktors aufgefangen und überschüssiges Phenol abdestilliert. Der Rückstand wurde in 5 bis 10 %iger Natronlauge gelöst und anschließend mit Schwefelsäure bei pH = 6,5 bis 7,0 Bis-(4-hydroxyphenyl)-sulfon ausgefällt. Nach Filtration und Trocknung erhielt man 145 g (58 %) Bis-(4-hydroxyphenyl)-sulfon in einer Reinheit von 98,5 %.

Beispiel 4

Man verfuhr wie in Beispiel 3 beschrieben, jedoch wurde das am Auslauf des Reaktors aufgefangene Reaktionsgemisch kontinuierlich auf den Festbettreaktor zurückgepumpt (Pumpleistung 2 l/h, Dauer 2 - 3 Stunden). Nach der wie in Beispiel 3 beschriebenen Aufarbeitung wurden 202,5 g (81 %) Bis-(4-hydroxyphenyl)-sulfon in einer Reinheit von 98,7 % erhalten.

Beispiel 5

Man verfuhr wie in Beispiel 4 beschrieben, jedoch wurde die Reaktion bei 160 °C und einem Druck von 700 bis 900 mbar durchgeführt. Nach der Aufarbeitung erhielt man 197,5 g (79 %) Bis-(4-hydroxyphenyl)-sulfon in einer Reinheit von 98,3 %.

Beispiel 6

Man verfuhr wie in Beispiel 4 beschrieben, jedoch füllte man den Reaktor mit gesinterten Borsäure-Kugeln (Durchmesser 3 bis 5 mm) anstelle von Glaskugeln. Nach der Aufarbeitung erhielt man 235 g (94 %) Bis-(4-hydroxyphenyl)-sulfon in einer Reinheit von 99,2 %.

Beispiel 7

Man verfuhr wie in Beispiel 5 beschrieben, jedoch füllte man den Reaktor mit gesinterten Borsäure-Kugeln anstelle von Glaskugeln. Nach der Aufarbeitung erhielt man 227,5 g (91 %) Bis-(4-hydroxyphenyl)-sulfon in einer Reinheit von 99,0 %.

**Ansprüche**

1. Verfahren zur Herstellung von Bis-(4-hydroxyphenyl)-sulfon durch Umsetzung von Phenol mit Schwefelsäure bei Temperaturen von 140 bis 230 °C, dadurch gekennzeichnet, daß man Phenol und Schwefelsäure im Molverhältnis von 1:1 bis 25:1 einsetzt und die Umsetzung in Abwesenheit eines inerten Lösungsmittels durchführt.

2. Verfahren nach Anspruch 1, dadurch gekennzeichnet, daß man die Umsetzung bei Temperaturen von 160 bis 200 °C durchführt.

3. Verfahren nach Anspruch 1, dadurch gekennzeichnet, daß man die Umsetzung in überschüssigem Phenol in Abwesenheit eines inerten Lösungsmittels durchführt und das überschüssige Phenol bei 160 bis 200 °C in und nach der Anfangsstufe oder in und nach der Zwischenstufe der Umsetzung fortschreitend abdestilliert oder nach im wesentlichen beendeter Reaktion das Phenol durch Destillation entzieht.

4. Verfahren nach Anspruch 1, dadurch gekennzeichnet, daß man Phenol und Schwefelsäure im Molverhältnis von 2,1:1 bis 25:1 verwendet.

5. Verfahren nach Anspruch 1, dadurch gekennzeichnet, daß man die Reaktion bei Drücken von 0,5 bis 1,5 bar durchgeführt.

6. Verfahren nach Anspruch 1, dadurch gekennzeichnet, daß man die Reaktion in Gegenwart eines Sulfonierungskatalysators durchführt.

Europäisches Patentamt

# EUROPÄISCHER RECHERCHENBERICHT

Nummer der Anmeldung

EP 90101450.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| X | PATENT ABSTRACTS OF JAPAN, unexamined applications, C Field, Band 10, Nr. 194, 8. Juli 1986 THE PATENT OFFICE JAPANESE GOVERNMENT, Seite 21 C 358 * Kokai-Nr. 61-36 253 (NIKKA CHEM IND) * -- | 1-5 | C 07 C 317/22<br>C 07 C 315/00 |
| X | PATENT ABSTRACTS OF JAPAN, unexamined applications, C Field, Band 6, Nr. 106, 16. Juni 1982 THE PATENT OFFICE JAPANESE GOVERNMENT, Seite 32 C 108 * Kokai-Nr. 57-35 559 (TOUKAI DENKA KOGYO) * -- | 1,6 | |
| A | DE - A1 - 2 804 080 (IMPERIAL CHEMICAL INDUSTRIES) * Ansprüche * -- | 1,2 | |
| | | | RECHERCHIERTE SACHGEBIETE (Int Cl 5) |
| A | DE - B - 1 618 023 (WITCO CHEMICAL COMPANY) * Anspruch * -- | 1,2 | C 07 C 317/00<br>C 07 C 315/00 |
| A | EP - A1 - 0 293 037 (AKZO N.V.) * Zusammenfassung * -- | 1 | |
| D,A | DE - A1 - 2 708 388 (KONISHI CHEMICAL INDUSTRY) * Anspruch * -- | 1 | |
| D,A | EP - A1 - 0 220 004 (AMOCO CORPORATION) * Zusammenfassung * ---- | 1 | |

Der vorliegende Recherchenbericht wurde für alle Patentansprüche erstellt.

| Recherchenort | Abschlußdatum der Recherche | Prüfer |
|---|---|---|
| WIEN | 09-05-1990 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 62